# EUROPEAN PATENT SPECIFICATION

(11) **EP 4 412 512 B1**
(45) Date of publication and mention of the grant of the patent: **04.06.2025**
(21) Application number: 22782855.5
(22) Date of filing: 07.09.2022
(51) Int. Cl.: A61B 5/00, G01J 3/44, G01N 21/65

(54) **RAMAN SPECTROSCOPY PROBE AND RAMAN SPECTROSCOPY APPARATUS**
RAMANSPEKTROSKOPIESONDE UND RAMANSPEKTROSKOPIEVORRICHTUNG
SONDE DE SPECTROSCOPIE RAMAN ET APPAREIL DE SPECTROSCOPIE RAMAN

(30) Priority: 07.10.2021 GB 202114356
(43) Date of publication of application: 14.08.2024
(73) Proprietor: Creo Medical Limited, Chepstow, Monmouthshire NP16 5UH (GB)
(72) Inventor: HANCOCK, Christopher Paul, Chepstow, Monmouthshire NP16 5UH (GB); BOOTON, Martin, Bath and North East Somerset BA5 3PZ (GB)
(74) Representative: Mewburn Ellis LLP
(86) International application number: PCT/EP2022/074806
(87) International publication number: WO 2023/057155

(56) References cited:
- WO-A1-2017/158331
- CN-A- 106 596 508
- US-A- 5 864 397
- US-A1- 2007 225 579
- US-A1- 2015 233 832
- US-A1- 2015 335 249
- US-A1- 2021 041 366
- US-B1- 6 614 523

## Description

### TECHNICAL FIELD

The invention refers to a Raman spectroscopy probe and, more particularly, to a Raman spectroscopy probe including an elongate body having a proximal end and a distal end, and at least one optical transmission line within the elongate body for guiding light for inducing Raman scattering in a tissue from the proximal end to the distal end and for guiding Raman scattered light from the distal end to the proximal end. The invention also refers to a Raman spectroscopy apparatus including the Raman spectroscopy probe and an analysis device.

### BACKGROUND

Raman spectroscopy can be used to identify different types of tissue of a human being or a mammal. In addition, it has been demonstrated that Raman spectroscopy can be used to identify cancerous tissue or other forms of tissue degeneration. This capability of Raman spectroscopy can also be used in endoscopic treatment of tissue for example for identifying the tissue to be treated.

US 8,175,423, US 6614523 and US 8,702,321 disclose an endoscopic Raman probe including a plurality of optical fibres for collecting Raman scattered light.

### SUMMARY

The invention is defined by the independent claims. The dependent claims describe preferred embodiments of the invention.

A Raman spectroscopy probe comprises an elongate body having a distal end and a proximal end, at least one optical transmission line within the elongate body, and a magnification layer arranged at or on the distal end and being exposable to tissue. The at least one optical transmission line is configured to guide light for inducing Raman scattering in a tissue from between the proximal end to and the distal end in a distal direction and for guiding Raman scattered light from between the distal end to and the proximal end in a proximal direction. The magnification layer has an exposed surface for contacting the tissue. The magnification layer is positioned such that the light for inducing Raman scattering impinges on the magnification layer. The magnification layer is for inducing surface-enhanced Raman scattering (SERS) at the exposed surface.

A Raman spectroscopy apparatus (or Raman spectroscopy unit) comprises the Raman spectroscopy probe described above and an analysis device including a Raman light source for generating monochromatic light (such as a laser that lases at a wavelength in the EM (electromagnetic) spectrum between infrared and ultraviolet light) and a spectrometer, wherein the at least one optical transmission line is connected to (e.g. in optical communication with) the analysis device, in particular connected to (e.g. in optical communication with) the monochromatic light source and/or the spectrometer.

Raman spectroscopy when used with a scoping device (e.g. an endoscope or the like) needs to ensure that sufficient Raman scattered light is collected at the distal end of the scoping device. This may be achieved by providing a large cross-sectional area of the at least one optical transmission line compared with the cross-sectional area of the scoping device. The invention proposes to additionally or alternatively enhance the ratio of Raman scattered light by using the principles of surface-enhanced Raman scattering. Thus, the invention suggests increasing the amount of light that is Raman scattered which results in increased sensitivity of the Raman spectroscopy.

Raman spectroscopy is a spectroscopic technique that can be used to analyse tissue by determining vibrational modes of molecules, although rotational and other low-frequency modes of systems may also be observed when incident EM energy is in the far infrared or even microwave/millimetre wave region. Raman spectroscopy provides a structural fingerprint by which molecules can be identified which then can be associated with a certain type of tissue and/or certain type of degeneration of tissue, for example exhibited by cancerous tissue.

Raman spectroscopy relies upon inelastic scattering of photons, known as Raman scattering. A source of monochromatic light, usually from a laser in the visible, near infrared, or near ultraviolet range is used. It may also be preferable to use longer wavelength light in the mid or far infrared region. The monochromatic light interacts with molecular vibrations, phonons, molecular rotations, or other excitations in the tissue, resulting in the energy of the laser photons being increased or decreased. The shift in energy gives information about the vibrational and/or rotational modes in the system.

The tissue to be analysed is illuminated with the monochromatic light which is transmitted by the at least one optical transmission line. Electromagnetic radiation from the illuminated tissue is collected and sent to an analysing device via the at least one optical transmission line. A monochromator may be provided in the analysing device. Elastically scattered radiation at the wavelength corresponding to the laser line (Rayleigh scattering) is filtered out by either a notch filter, edge pass filter, or a band pass filter, while the rest of the collected light is dispersed onto a detector of the analysing device.

The at least one optical transmission line may include at least one collection fibre for guiding light from the distal end to the proximal end and/or at least one illumination fibre for guiding light from the proximal end to the distal end. The illumination fibre may also be used to deliver the wideband white light or narrow band light for direct vision as well as Raman spectroscopy. In this arrangement, an optical switch or an optical coupler may be used to deliver either the monochromatic laser light for Raman or the wideband light from say a halogen light bulb for direct vision. Details will be described later.

The at least one illumination fibre can be configured to guide light for inducing Raman scattering in a tissue between the proximal end and the distal end in a distal direction. The at least one collection fibre can be configured to guide Raman scattered light between the distal end and the proximal end in a proximal direction. The distal direction is the direction from the proximal end towards the distal end. The proximal direction is the direction from the distal end towards the proximal end. Optionally, the optical transmission line is configured to guide light for inducing Raman scattering in a tissue from the proximal end to the distal end and for guiding Raman scattered light from the distal end to the proximal end.

The optical transmission line may include a double-clad fiber which is a class of optical fiber with a structure consisting of three layers of optical material instead of the usual two. The double-clad fiber includes an inner-most layer, an inner cladding, and an outer cladding. The inner-most layer can act as a core similar to the core of a standard optical fiber having two layers. The inner-most layer is surrounded by the inner cladding, which is surrounded by the outer cladding. The three layers are made of materials with different refractive indices. The inner cladding has a higher refractive index than the outer cladding. The core may correspond to the illumination fibre and can be used for guiding light for inducing Raman scattering in a tissue. The inner cladding may be used as the collection fibre for guiding light from the distal end to the proximal end. The inner cladding may have a higher numerical aperture compared to the core providing a good collection of Raman scattered light. The comparatively low numerical aperture of core allows to direct the light onto a small area of the tissue.

The Raman spectroscopy probe may be connected to the analysis device. The analysis device may include a monochromatic Raman light source, such as one or more lasers, for illuminating the tissue to be analysed. The Raman light source may be connected to (e.g. in optical communication with) the at least one optical transmission line, in particular connected to (e.g. in optical communication with) the at least one illumination fibre.

The analysis device may include components for analysing the Raman scattered light emitted by the tissue which was subjected to the illumination with the light emitted by the Raman light source. For example, the analysis device may include filters, such as a notch filter, edge pass filter, or a band pass filter, for filtering the Rayleigh scattered light from the light feedback to the analysis device via the one optical transmission line, preferably via at least one collection fibre. The analysis device may include a spectrometer for determining the intensity and/or the wavelength of the Raman scattered light. The spectrometer may include means for dispersing the light to be analysed and an optical detector for detecting the light, such as CCD detector. The means for dispersing the light to be analysed may spatially separate light depending on the wavelength and may include a grating.

The at least one collection fibre may be coupled to (e.g. in optical communication with) the spectrometer and/or the at least one illumination fibre is coupled to (e.g. in optical communication with) the Raman light source.

The Raman spectroscopy probe may be used as a scoping device or in conjunction with a scoping device. For example, the Raman spectroscopy probe may be inserted into a catheter or other type of scoping device such as an endoscope, bronchoscope, cystoscope, nephroscope, arthoscope, colonoscope, or laparoscope. In addition, the Raman spectroscopy probe may be used as an endoscope, bronchoscope, cystoscope, nephroscope, arthoscope, colonoscope, or laparoscope.

Generally speaking, surface-enhanced Raman spectroscopy or surface-enhanced Raman scattering (SERS) is a surface-sensitive technique that enhances Raman scattering by molecules adsorbed on a surface such as a rough metal surfaces or nanostructures such as plasmonic-magnetic silica nanotubes. The enhancement factor can be as much as 10⁶ to 10⁹. In other words, this phenomenon can be regarded as a significant resonant enhancement of the Raman scattering from molecules adsorbed at the surface.

With the invention, the magnification layer is arranged at or on the distal end and is exposed to the tissue. The magnification layer may be attached to the distal end of the Raman spectroscopy probe and exposed such that the magnification layer can be brought in contact with that issue to be analysed. The exposed part of the magnification layer can be considered the exposed surface. In other words, the exposed surface of the magnification layer can be brought in contact with the tissue. In this way, the molecules of the tissue "adsorb" to the magnification layer resulting in SERS. In other words, the magnification layer provides the giant resonant enhancement of the Raman scattering from molecules. Thus, the Raman scattering can be greatly enhanced resulting in an increased sensitivity of the Raman spectroscopy.

The magnification layer has physical properties which allow the generation of surface plasmons which are thought to induce the surface enhanced Raman scattering. The material and other properties of the magnification layer allow the generation of surface plasmons which induce SERS.

The magnification layer may have a surface roughness on the nano scale, i.e. the surface has a texture whose dimensions are in the nanometre range. This nanometre range roughness is thought to provide peaks in the electric field generated by the surface plasmons which (resonantly) couple with the vibrations in the molecules increasing the amount of Raman scattered light.

The magnification layer is arranged such that it can be brought in contact with the tissue to be analysed. For example, the magnification layer is arranged on an outer or exposed surface of the Raman spectroscopy probe. At the same time, the magnification layer is arranged at such a position that the light for inducing Raman scattering, for example light that is transmitted by the illumination fibre, impinges on the magnification layer. This means that the tissue to be analysed is both in contact with the magnification layer and illuminated by the light for inducing Raman scattering. The magnification layer can be a part of the outer surface of the Raman spectroscopy probe.

For example, the light for inducing Raman scattering may propagate through the magnification layer. However, other positions of the magnification layer are possible. For example, the light for inducing Raman scattering propagates through the tissue and then impinges on the magnification layer.

The magnification layer may be attached to a component that can be releasably attached to the Raman spectroscopy probe. For example, the magnification layer may be arranged on a disc or a lens (structure) that can be releasably attached to the distal end of the Raman spectroscopy probe.

The distal end of the Raman spectroscopy probe is positioned for Raman spectroscopy such that the magnification layer is in contact with the tissue to be analysed. For example, the magnification layer is pressed against the tissue to be analysed. Then, the tissue to be analysed is subjected to the light for inducing Raman scattering. The Raman scattered light is collected and analysed.

The elongate body of the Raman spectroscopy probe may define the outer dimensions of that part of the Raman spectroscopy probe that is inserted into a cavity of a human being or a mammal, a catheter or other type of scoping device. The elongate body may provide the endoscopic capabilities of the Raman spectroscopy probe.

The elongate body and all components arranged within the elongate body may be flexible enough to be guided through an elongated cavity of the human being or the mammal to be treated. For example, this elongated cavity of the human body may be the gastrointestinal tract or the bronchial tree of the lungs that contains bends and twists. In other words, the elongate body and all components arranged within the elongate body may be flexible in order to permit the distal end of the Raman spectroscopy probe. The target site may be a cavity (or zone or region) or part of a cavity (or zone or region) in the body whose tissue is to be analysed, treated and/or imaged.

Furthermore, the outer surface of the elongate body may be made from such a material such that it can be sterilised or subjected to cleaning routines necessary for surgical procedures.

The Raman spectroscopy probe has an elongated structure which can be (solely) constituted by the elongate body. The distal end of the elongate body is that end of the elongated structure of the Raman spectroscopy probe which is to be inserted into a patient. The proximal end of the elongate body is preferably connected to the analysis device or other devices to which the elongate instrument can be connected to, and may in use remain outside the patient. The elongate body may define the distal end to the proximal end of the Raman spectroscopy probe.

The Raman spectroscopy probe and/or the elongate body may have a rod-like structure. The Raman spectroscopy probe and/or the elongate body are flexible perpendicular to its direction of extension (corresponding to a longitudinal direction) and rigid in its directions of extension.

The elongate body may have a tubular structure and may include a sheath for insulating or shielding (e.g. protecting) other components of the Raman spectroscopy probe from the outside, such as fluids. The sheath may be a lubricious polymer sheath for reducing friction, for example when inserted into a scoping device and/or with surrounding tissue.

The elongate body may further comprise a strain relief layer to limit the bending of the at least optical transmission line. The strain relief layer may be covered by the sheath described above. The strain relief layer could include a braid, a coil, and/or a tube made of a polymer or metal wire.

The Raman spectroscopy probe may include an instrument lumen that is a passageway arranged within the elongate body of the Raman spectroscopy probe. Preferably, the instrument lumen completely extends from the distal end to the proximal end. At least a part of an elongate instrument can be arranged or placed within the instrument lumen. Due to the elongated structure of the elongate body and, thus, of the instrument lumen, an elongated part of the elongate instrument can be placed or arranged within the instrument lumen. For example, a distal end of the elongate instrument coincides or is arranged near a region at the distal end of the elongate body. A proximal end of the elongate instrument may extend beyond the proximal end of the elongate body.

The instrument lumen may be defined by a wall or surface which surrounds the instrument lumen from the distal end to the proximal end. The instrument lumen may be separated from the remaining space within the elongate body in a fluid tight manner. The wall may be constituted by a hollow tube arranged within the elongate body.

It is also possible that the instrument lumen is defined by the components arranged within the elongate body such as the at least one illumination fibre, the at least one collection fibre and a filler material placed between the fibres. In this case, no separate wall may be present for providing the instrument lumen.

The least one optical transmission line includes an optical fibre, such as a single-mode fibre or a multi-mode fibre. The at least one illumination fibre may be called an excitation fibre and is configured to transmit light, in particular laser light, from the proximal end to the distal end. Optionally, the at least one illumination fibre extends beyond the proximal end of the elongate body and is connected to a Raman light source such as a laser.

The Raman light source (or excitation light source) may be configured to generate monochromatic light having a wavelength/frequency which coincides with the excitation frequency of the molecule to be analysed using Raman spectroscopy. The monochromatic light may be light having a single wavelength or the wavelength range is narrow. For example, the wavelength is in the visible spectrum, such as 785 nm with 100 mW of power. However, longer wavelengths (lower frequencies) down to far infrared frequencies that also sit in the THZ or mm-wave band may be used. Far infrared (FIR) is a region in the infrared spectrum of electromagnetic radiation. Far infrared is often defined as any radiation with a wavelength of 15 micrometres (µm) to 1 mm (corresponding to a range of about 20 THz to 300 GHz).

The Raman light source generates light having a wavelength in the ultra-violet spectrum (for example 244 nm, 257 nm, 325 nm, 364 nm), in the visible spectrum (for example 457 nm, 473 nm, 488 nm, 514 nm, 532 nm, 633 nm, 660 nm), in the near infra-red spectrum (for example 785 nm, 830 nm, 980 nm, 1064 nm) or up to the mid infrared (IR) spectrum or far IR spectrum.

The distal end of the at least one illumination fibre may be treated in such a way that light can be emitted from the distal end of the at least one illumination fibre. For example, the distal end surface of the at least one illumination fibre may be inclined to the extension of directions of extension. The angle of inclination may be 90° or less. Alternatively or additionally, the distal end surface of the at least one illumination fibre may be connected to a lens structure. Both options may be provided for directing the light emitted from the at least one illumination fibre to a part of the target area.

The at least one collection fibre is configured to transmit light, in particular Raman scattered light from the target site, from the distal end to the proximal end. The distal end of the at least one collection fibre may be treated in such a way that light can be collected, i.e. coupled into the distal end of the at least one collection fibre. For example, the distal end surface of the at least one collection fibre may be inclined to the extension of directions of extension. The angle of inclination may be 90° or less. Alternatively or additionally, the distal end surface of the at least one collection fibre may be connected to a lens structure. Both options may be provided for collecting as much as Raman scattered light from the illuminated part of the target area and for coupling the Raman scattered light into the at least one collection fibre. The at least one collection fibre may be connected to the analysis device, in particular to the spectrometer.

Optionally, a plurality of optical transmission lines, in particular a plurality of illumination fibres and/or a plurality of collection fibres, may be provided. The at least one collection fibre and at least one illumination fibre may be fixed to each other. For example, the space between the at least one illumination fibre and the at least one collection fibre may be filled with an adhesive and/or other filler material.

The at least one optical transmission line may be arranged between the instrument lumen and the elongate body. In other words, the at least one optical transmission line may be arranged between the outer surface of the instrument lumen and an inner surface of the elongate body. For example, the at least one optical transmission line may be arranged in a space defined by the wall surrounding the instrument lumen and the elongate body.

The arrangement of the plurality of optical transmission lines may be such that the space defined by the plurality of optical transmission lines is not arranged side-by-side to the instrument lumen in a cross-sectional view. For example, in a cross-section view, there may be filler material between the plurality of optical transmission lines and the instrument lumen. Further, the space defined by the plurality of optical transmission lines may have a shape that differs from the shape of the instrument lumen in a cross-section view. Worded differently, the space defined by the plurality of optical transmission lines does not follow the perimeter of the lumen. For example, the instrument lumen and the space defined by the plurality of optical transmission lines may have a circular or rectangular cross-section and are located spaced away from each other.

Optionally, at least a part of the plurality of optical transmission lines is in contact with the instrument lumen. Thus, the remaining optical transmission lines are arranged between the elongate body and a plurality of optical transmission lines in contact with the instrument lumen. For example, in a cross-sectional view, the plurality of optical transmission lines may be arranged in one or more sections which are arranged between the instrument lumen and the elongate body. In particular, one or more sections follow the shape of the instrument lumen. In other words, in a cross-sectional view, the outer shape of the one or more sections is aligned with the shape of the instrument lumen (e.g. each section may be a circumferential section and may have a generally arc-shaped form).

The plurality of optical transmission lines may be arranged in such a way that they are in contact with the instrument lumen. For example, the plurality of illumination fibres and collection fibres may define one or more sections (e.g. circumferential sections) of the surface of the instrument lumen in a cross-sectional view of the elongate body.

What is more, the plurality of optical transmission lines may completely fill a sector between the instrument lumen and elongate body. For example, the sector is defined by a part of the outer surface of the instrument lumen, a part of the inner surface of the elongate body, and lines connecting the ends of the part outer surface of the instrument lumen with the ends of the part of the inner surface of the elongate body.

In an optional embodiment, the Raman spectroscopy probe further comprises a plurality of optical transmission lines, in particular a plurality of illumination fibres and collection fibres, wherein, in a cross-sectional view of the elongate body, the plurality of optical transmission lines is distributed around the circumference of the instrument lumen, wherein preferably the plurality of optical transmission lines is distributed around the majority or entirety of the circumference of the instrument lumen and, wherein further preferably the plurality of optical transmission lines forms a ring of fibres.

The plurality of illumination fibres and collective fibres may be distributed around the circumference of the instrument lumen in such a way that the illumination fibres and collection fibres do not contact each other. The space between the illumination fibres and collection fibres may be filled by an adhesive or a filler material which may contribute to the definition of the instrument lumen. Alternatively, the instrument lumen is defined by a wall whereby the plurality of illumination fibres and collection fibres may be in contact with the wall. In these cases, further illumination fibres and/or collection fibres may be provided which are spaced from the instrument lumen by the plurality of collection fibres and illumination fibres described above and defining or participating in the definition of the instrument lumen.

The plurality of illumination fibres and collection fibres may be arranged in one or more sections around the circumference of the instrument lumen or can completely surround the circumference of the instrument lumen. As discussed, the plurality of illumination fibres and collection fibres may be tightly packed such that they contact each other or can be spaced apart from each other, for example by a filler material.

The arrangement not in contact with each other may reduce cross-coupling of light between the individual fibres. However, it is possible that the plurality of optical transmission lines may be covered by an optically non-transparent material in order to reduce or eliminate cross-coupling of light between individual fibres. **In** this case, the plurality of illumination fibres and collection fibres may be in contact with each other, in particular the optically non-transparent cover materials of the optical transmission lines contact each other.

**In** a cross-sectional view, a line intersecting the plurality of illumination fibres and collection fibres may extend around the circumference of the instrument lumen, either completely or at least a section thereof. The line intersecting the plurality of illumination and collection fibres may not intersect with the instrument lumen in a cross-sectional view.

The elongate body and/or the instrument lumen may have a circular, rectangular, oval or ellipsoid shape in a cross-sectional view. The plurality of illumination fibres and collection fibres which are arranged in contact with the instrument lumen may follow the circular, rectangular, oval or ellipsoid shape of the instrument lumen such that at least a part of the plurality of illumination fibres and collection fibres are arranged in a circular, rectangular, oval or ellipsoid shape in a cross-sectional view of the elongate body. Thus, at least a part of the plurality of illumination fibres and collection fibres are arranged parallel to the shape of the instrument lumen in a in a cross-sectional view of the elongate body.

The arrangement of the plurality of illumination fibres and collection fibres in form of a ring may be constituted in that the plurality of collection fibres and illumination fibres contact each other along the circumference of the instrument lumen. Depending on the shape of the instrument lumen in a cross-sectional view, the ring of collection fibres and illumination fibres may have a shape in a cross-sectional view which is identical to the shape of the instrument lumen. Preferably, the instrument lumen has a circular shape in a cross-sectional and the ring of collection fibres and/or illumination fibres may be circular in a cross-sectional view.

The provision of the plurality of illumination fibres and collection fibres around the circumference of the instrument lumen helps to reduce a cross-sectional area of the elongate body in an advantageous manner: the plurality of illumination fibres and collection fibres are arranged close to the perimeter of the elongate body providing sufficient space for arranging a high number of collection fibres and illumination fibres since the plurality of fibres are arranged radially outside. The instrument lumen is arranged radially inside. In this way, for a given diameter of the radius of the elongate body and of the instrument lumen, the space is advantageously allocated compared to a way where the instrument lumen and the plurality of collection fibres and illumination fibres are arranged side-by-side.

The increased proportion of Raman scattered light induced by the magnification layer allows to reduce the number and/or cross section of the optical transmission lines, preferably the collection fibres since less Raman scattered light needs to be collected. This allows to reduce the diameter of the Raman spectroscopy probe and/or to include the instrument lumen with reducing the sensitivity of the Raman spectroscopy measurements.

In an optional embodiment, in a cross-sectional view of the elongate body, the plurality of illumination fibres is arranged in a first ring of fibres and a plurality of collection fibres are arranged in a second ring of fibres coaxially arranged with the first ring of fibres.

The second ring of collection fibres may surround the first ring of illumination fibres; i.e. the plurality of collection fibres is arranged radially outside of the plurality of illumination fibres. In this way, more collection fibres may be provided compared to the number of illumination fibres. As stated previously, more Raman scattered light can be collected if the number of collection fibres is increased. Thus, this arrangement provides a tightly packed arrangement of collection fibres and illumination fibres resulting in a high number of illumination as well as collection fibres. What is more, the illumination fibres and collection fibres may be easily arranged within the elongate body. For example, the ring of illumination fibres is provided and then, subsequently, the ring of collection fibres.

The plurality of illumination fibres and collection fibres may be in contact with each other. The first ring of illumination fibres may be separated from the second ring of collection fibres by a middle wall, or another structural component arranged within the elongate body. However, it is also possible that first ring of illumination fibres may be in contact with the second ring of collection fibres.

The instrument lumen may be arranged within the first ring of illumination fibres. In a cross-sectional view of the elongate body, the instrument lumen, the first ring of illumination fibres, the second ring of collection fibres, and/or the elongate body may be coaxially arranged.

In an optional embodiment, a longitudinal axis of the elongate body is coaxial with a longitudinal axis of the instrument lumen. Optionally, the longitudinal axis of the elongate body coincides with the longitudinal axis of the instrument lumen.

In a cross-sectional view of the elongate body, a centre of the elongate body is coaxially arranged with a centre of the instrument lumen. In addition, the centre of the elongate body or the centre of the instrument lumen may be coaxially arranged to the ring or rings of fibres.

The longitudinal axis may extend along the centre of the elongate body or the instrument lumen. The centre may correspond to the centre of mass and/or the geometric centre. For example, the centre may be a circle centre if the instrument lumen and/or the elongate body are circular in a cross-sectional view.

In an optional embodiment, the instrument lumen is configured to slidably receive the elongate instrument. That is, the elongate instrument is separable and detachable from the Raman spectroscopy probe. In another embodiment, the Raman spectroscopy probe includes the elongate instrument, wherein the elongate instrument is fixedly arranged in the instrument lumen such that it is not separable or detachable from the probe.

The elongate instrument may be an endoscopic instrument configured to be inserted into and/or pulled out the instrument lumen. For example, different elongate instruments may be inserted into the instrument lumen. In particular, it is possible to push the elongate instrument out of the distal end of the elongate body, for example to use the elongate instrument at the target site while the elongate instrument remains within the instrument lumen as long as the Raman spectroscopy probe is navigated to the target site.

In this case, the instrument lumen may be defined by a wall and/or hollow tube. The wall and/or the hollow tube may be configured as a fluid tight seal to seal the Raman spectroscopy probe. The inner diameter of the instrument lumen may be slightly bigger than the outer diameter of the elongate instrument such that the elongate instrument can be moved within the instrument lumen.

In another embodiment, the elongate instrument is fixed to the instrument lumen. In this case, the elongate instrument is a unitary component with the elongate body. Thus, the Raman spectroscopy probe may be used for obtaining Raman spectroscopy measurements and for utilising their capabilities of the elongate instrument. The Raman spectroscopy probe therefore has additional functionalities or capabilities.

In another embodiment, the Raman spectroscopy probe does not include an instrument lumen. For example, the Raman spectroscopy probe solely includes a plurality of optical transmission lines, i.e. at least one illumination fibre and at least one collection fibre which can be arranged as described above. The at least one illumination fibre and/or at least one collection fibre may be arranged instead of the instrument lumen. The illumination fibres and collection fibres of the Raman spectroscopy probe may be arranged in a tightly packed bundle surrounded by the elongate body as described above.

The elongate instrument may be a needle, a scalpel or any other surgical instrument needed to treat the tissue at the target site. The surgical instrument has outer dimensions such that it can be inserted into the instrument lumen; the surgical instrument may be an endoscopic instrument.

In an optional embodiment, the elongate instrument is a surgical instrument, an endoscopic instrument for modifying tissue, and/or elongate camera.

The endoscopic instrument may be an electrosurgical instrument for ablating, coagulating, cutting and/or stimulating tissue by the emission of electromagnetic radiation, in particular radio frequency radiation and/or microwave radiation. The elongate camera may be used for imaging the tissue which is treated by the radiation emitted by the endoscopic instrument or which is analysed using Raman spectroscopy.

The term "electrosurgical" is used in relation an instrument, apparatus or tool which is used during surgery and which utilises microwave and/or radiofrequency electromagnetic (EM) energy.

The elongate instrument may be slidably inserted into the instrument lumen such that it can be pushed or pulled out of the distal end for imaging/treating the tissue to be analysed. Thus, it is possible to simultaneously insert the device for ablating/cutting tissue with the analysis device for Raman spectroscopy. This simplifies the insertion process as various functionalities can be simultaneously inserted.

The endoscopic or elongate camera may include an image capturing device. The image capturing device is configured to create a series of the electrical signals based on optical image which is projected on an optical sensor arranged within the image capturing device. The optical sensor may be arranged at or close to a distal end of the elongate camera. A wire may extend within the elongate camera from image capturing device (in particular the optical sensor) to a proximal end of the elongate camera for transmitting the electrical signals. The proximal end of the elongate camera is connected to a display device. The display device may include a processor to process the series of electrical signals generated by the image capturing device. In particular, the processor may generate an optical image that can be displayed by a display of the display device.

In an optional embodiment, the at least one optical transmission line includes a distal end surface, wherein the magnification layer is arranged on the distal end surface.

The distal end surface may be transparent to the light for inducing Raman and/or the Raman scattered light. The light for inducing Raman scattering may be guided through the distal end surface. The distal end surface may cover the at least one illumination fibre and/or the at least one collection fibre. The distal end surface may be the distal end surface of the elongate body. In other words, the distal end surface may close or seal the elongate body at the distal end.

The distal end surface may be positioned at the distal end of the elongate body. The distal end surface may be an outer surface of the elongated body; the distal end surface may be a surface which is exposed to the tissue, i.e. can be brought in contact with the tissue to be analysed. The magnification layer may cover the complete distal end surface or only parts thereof. The distal end surface and the sheath may completely define the outer surface of the elongate body that can be inserted into the cavity of the patient. A lens structure may be arranged between the distal end surface and the distal end of the at least one optical transmission line.

The distal end surface may be a substrate on which the magnification layer is arranged. For example, the magnification layer is deposited on the distal end surface. The distal end surface may be permanently fixed to the elongate body or the distal end surface may be releasably fixed to the elongate body. For example, the distal end surface is removed during cleaning of the elongated body in order not to destroy the magnification layer.

In an optional embodiment, the distal end surface includes a disc for closing the distal end.

The disc may be made from an optically transparent material. The disc has an outer shape which corresponds to the shape of the elongated body in a cross-sectional view thereof. For example, the disc has a circular shape. The disc may provide the distal end of the Raman spectroscopy probe. The magnification layer may be deposited or arranged on the disc.

In an optional embodiment, the distal end surface is a distal end of the at least one illumination fibre. Thus, the magnification layer is directly attached to the distal end of the at least one illumination fibre. Preferably, all illumination fibres are provided with the magnification layer. In is this embodiment, the Raman spectroscopy probe may not include the disc, but distal ends of the plurality of illumination fibres and/or of the plurality of collection fibres define a distal end surface of the Raman spectroscopy probe.

In an optional embodiment, the Raman spectroscopy probe further comprises a lens structure arranged at the distal end for focusing light on the issue and/or for focusing light from the issue into at least one optical transmission line, wherein the magnification layer is arranged on the lens structure.

The lens structure may be for focussing the light from the optical transmission line onto tissue contacting the exposed portion and/or for focussing the Raman scattered light from tissue contacting the exposed portion into the optical transmission line.

The lens structure may comprise one or more lenses or other optical components for guiding, focusing and/or redirecting light. The lens structure may form the distal end surface of the Raman spectroscopy probe and may be arranged instead of the disc. The disc may be considered a lens structure which does not affect the direction of propagation of light therethrough.

The lens structure may be used to focus light that is guided by the illumination fibre onto the tissue to be analysed and/or to focus/collect the light that is Raman scattered by the tissue into the collection fibres. The lens structure may further include optical filters.

The magnification layer may be arranged on an outer surface of the lens structure. The lens structure may be a substrate on which the magnification layer is deposited. The magnification layer may cover the complete outer surface of the lens structure or only parts thereof.

**In** an optional embodiment, the magnification layer is positioned in line with or in optical communication with the at least one illumination fibre. Further optionally, the magnification layer is positioned such that the magnification layer is solely in line with or in optical communication with the at least one illumination fibre.

**In** other words, the magnification layer covers an area of the disc or the lens structure which corresponds to the cross-sectional area of the at least one illumination fibre, preferably all illumination fibres. This means that the light for inducing Raman scattering is guided through the magnification layer. This arrangement provides that the tissue to be analysed is both in contact with the magnification layer and illuminated by light for inducing Raman scattering.

**In** an optional embodiment, the magnification layer is (solely) positioned in line with or in optical communication with the at least one illumination fibre. This means that the magnification layer does not cover the collection fibre such that light that is Raman scattered back from the tissue does not propagate through the magnification layer. This avoids attenuation of the Raman scattered light by interaction with the magnification layer. The magnification layer may cover the complete distal end surface (e.g. the disc or the lens structure) except for the areas which overlap with the at least one collection fibre.

The optical structure or the disc may be an optically transparent component through which the light from the illumination fibre can reach the target area and/or the Raman scattered light can couple into the collection fibre. The at least one illumination fibre and/or the at least one collection fibre may be fixed to the disc or lens structure such that the fibres cannot be moved relative to the disc or lens structure. The disc or lens structure may additionally be configured to seal the distal end of the elongate body. The lens structure may be dome shaped.

In an optional embodiment, the disc or the lens structure include an aperture which is aligned with the instrument lumen. This allows the elongate instrument to be moved out of the distal end of the elongate body.

In an embodiment, the magnification layer, optionally the exposed surface of the magnification layer, is made is made from an electrically conductive material, such as metal, and includes a surface structure on a nanoscale for inducing surface-enhanced Raman scattering.

The enhancement of the Raman signal by an electrically conductive nano-structured surface is considered to be caused by the combined action of two main effects, the so-called electromagnetic mechanism and chemical mechanism. The electromagnetic enhancement occurs due to the action of the near optical field, amplified in the vicinity of the nanostructure surface as a result of the resonant excitation of surface plasmon oscillations in electrically conductive or metallic clusters, on the molecules under investigation. The parameters of the resonant excitation of plasmons and the scale of enhancement of the near optical field depend on the nanostructure morphology, parameters characterizing the conductivity of the electrically conductive material (metal), and the properties of the dielectric environment. The chemical mechanism may contribute to SERS for adsorbate molecules that are in direct contact with the electrically conductive surface. This mechanism results from the coupling of the molecular electronic orbitals with the conduction band states at the electrically conductive surface and is called the chemical enhancement effect. Chemical enhancement is not universal: it depends on the nature of the molecule and its chemical affinity to the metal surface.

The surface structure (the roughness) of the magnification layer may be chosen such that effects of the electromagnetic mechanism and/or the chemical mechanism are high with typical tissues to be analysed using the Raman spectroscopy probe.

The term "surface structure on a nanoscale" means that the parameters used to describe the surface roughness of the magnification layer are in the range between 1 nm and 500 nm, preferably between 5 nm and 150nm. The magnification layer may have a mean or average line around which the exact surface varies. The variation of the surface around the mean or average line is in the nanometre range.

The magnification layer may have a periodical surface structure. Alternatively, the surface structure of the magnification layer is statistically random.

In an optional embodiment, the magnification layer includes material patches which are distributed over the magnification layer.

A material patch (or an island) is an accumulation of material which includes at least 5 to 10 atoms of the material of the magnification layer. A material patch is optionally completely separated or spaced apart from other material patches. Thus, material that is in contact with each other can be regarded a single material patch. Material that is in contact with each other forms a single material patch.

A material patch may be considered one or more nanoparticles (if they are in contact with each other) attached to a substrate. The plurality of nanoparticles forms the magnification layer. A material patch may have a defined shape and structure and is intentionally positioned with respect to other material patches. However, the material patches may be statistically (evenly) distributed over the magnification layer. This means that the distances between two adjacent material patches are statistically (evenly) distributed. In other words, the distribution of the material patches over the magnification layer is approximately even - the material patches are not arranged in particular areas of the magnification layer. The density of the material patches is approximately even throughout the magnification layer.

The material patches may have identical shapes to each other. However, it is also possible that the material patches differ from each other. For example, the manufacturing of the material patches includes depositing doses of material while not exactly controlling the generated shape of the material patches. The material patches may be variations of a common shape wherein the variations can be artefacts of the manufacturing process. Therefore, it is possible to attribute an average shape to the material patches. In short, the shape and/or the position of the material patches may only be determined using statistics, for example by providing an average value and a variation (variance) thereof. These statistical distributions of positions and/or the shape of the material patches provide the surface roughness of the magnification layer.

In an optional embodiment, dimensions of the material patches and dimensions between the material patches are in the same order of magnitude.

Dimensions of the material patches refer to the width, length and/or height. Dimensions between the material patches refer to the distance between two adjacent material patches and can be used to characterise how densely material patches are packed within the magnification layer. These two types of dimensions may be statistically (evenly) distributed.

The dimensions between the material patches and the dimensions of the material patches may be in the nanometre range. For example, the average value of the dimensions of the material patches may be greater than the average value of the dimensions between the material patches. This indicates that the magnification layer includes more material than holes. In this optional embodiment, the magnification layer may provide increased Raman scattering while being less transparent to light for inducing Raman scattering. However, it is also possible that the average value of the dimensions of the material patches is smaller than the average value of the dimensions between the material patches. This indicates that the magnification layer includes more holes than material patches. In this optional embodiment, the magnification layer may provide less increase of the Raman scattering compared to the situation described above. However, due to the more coarsely distributed material patches, the magnification layer allows more propagation of light there through.

It is also possible to precisely control the position of the material patches and/or the shape of the material patches during manufacture of the magnification layer. This allows to provide a magnification layer in which the material patches are approximately positioned in a grid like structure and/or have the approximately same shape. The arrangement of the material patches may be described as being a periodic structure whose periodicity may be defined using unit cells. The unit cell may be rectangular, square-shaped, or other shapes which are capable of providing a periodic structure of material patches.

In an optional embodiment, the material patches have a width between 20 nm to 150nm and/or a height between 10 nm to 120nm, wherein optionally a distance of between two adjacent material patches is between 10 nm to 120nm.

The width may be the longest distance between any two points on a perimeter of the material patches, the distance extending parallel to the extension of the magnification layer. For example, if the material patches have a circular cross section, the width may correspond to the diameter of the material patches. There may be two main distances between two adjacent material patches, however both are in the above-described range. For example, if the unit cell of the periodic structure of the material patches is a rectangle, there are two distances to describe the distance between two adjacent material patches.

The above-described values may refer to average values or be values to describe the periodicity of the material patches.

In an optional embodiment, the material patches are identical to each other, wherein optionally the material patches are dome shaped, preferably have the shape of a spherical dome.

Other shapes of the material patches are also possible such as pyramids. Furthermore, the identical material patches may be positioned in a periodical ordering. Optionally, the dome-shaped material patches have a diameter of approximately 80 nm, height of approximately 40 nm, and/or a distance to an adjacent material patch of approximately 40 nm. The unit cell may be a square.

In an optional embodiment, the magnification layer is a continuous material layer including thick portions defining protrusions and thin portions defining recesses, wherein the thin portions have a thickness allowing the transmission of the light for inducing Raman scattering.

The above-described material patches may be considered corresponding to the protrusions/thick portions of the continuous magnification layer whereby the recesses/thin portions have a thickness of zero. Thus, the above-described remarks on the material patches equally apply to the protrusions/thick portions and recesses/thin portions. For example, the recesses extend along parallel lines. The recesses may form a grid structure. Two recesses can be perpendicular to each other. For example, the recesses completely surround a protrusion. However, it is also possible that the protrusions and recesses extend parallel to each other.

The protrusions and/or the recesses have a width that is significantly smaller than the wavelength of the light propagating through the magnification layer. This allows to reduce diffraction phenomena happening at the protrusion/recess structure, especially if the magnification layer includes a periodic structure.

The protrusions/thick portions and/or the recesses/thin portions may not have a constant thickness but a varying thickness. The varying thickness of the protrusions can increase the surface roughness of the magnification layer. The thickness of the recesses/thin portions may be so little at certain points/areas that light can propagate through these parts of the recesses. For example, the recesses/thin portions have a central portion which has a thickness allowing the propagation of light there through. This central portion may be positioned in the middle between the highest points/areas of the protrusions.

In an optional embodiment, the protrusions or thick portions have a height between 10 nm to 120nm and/or the recesses or thin portions have a thickness between 1 nm and 40 nm.

The height of the protrusions/thick portions may alternatively be regarded as the thickness/thick portions of the protrusions. The height of the protrusions may correspond to the greatest thickness of the protrusions. The thickness of the recesses/thin portions may correspond to the smallest or the greatest thickness of the recesses/thin portions. The height and/or the thickness may be an average value all values of the periodic arrangement of the protrusions and recesses. The height and/or thickness may be measured starting from the substrate on which the magnification layer is arranged and ending with the point of the recess/thin portion and/or protrusion/thick portion that is furthest away from the substrate.

Optionally, the protrusions/thick portions have height of approximately 80 nm, a peak (of the protrusion) to trough distance (of the recess/thin portion) in the height direction of approximately 60 nm (resulting in a thickness of the recess/thin portion of approximately 20 nm), and/or a peak (of the protrusion) to trough distance (of the recess) in the direction of the extension of the magnification layer of approximately 60 nm.

In an optional embodiment, the magnification layer includes a metal, optionally gold, silver, copper, and/or aluminium. The above-described materials may be used for the material patches, the protrusions and/or the recesses. The above-described materials are considered to provide a great enhancement of Raman scattering in the tissue due to SERS.

In an optional embodiment, the disc or the lens structure includes an optically transparent material, such as fused silica, magnesium fluoride, glass (amorphous silica), and/or sapphire.

In this specification "microwave" may be used broadly to indicate a frequency range of 400 MHz to 100 GHz, but preferably the range 1 GHz to 60 GHz. Preferred spot frequencies for microwave EM energy include: 915 MHz, 2.45 GHz, 3.3 GHz, 5.8 GHz, 10 GHz, 14.5 GHz and 24 GHz. 5.8 GHz may be preferred.

The terms "connected" and "coupled" are used herein in an open sense, in that, when one element is said to be connected/coupled to another element, it is to be understood that the two elements may be directly connected/coupled together such that there are no intervening elements and also that the two elements may be indirectly connected/coupled together such that there are one or more intervening elements. However, when two elements are said to be "directly connected" or "directly coupled" together, it is to be understood that there are no intervening elements between them.

The term "outer diameter" (or the "maximal outer diameter") corresponds to the longest distance between any two points on the perimeter of the elongate body (or any other component to which the outer diameter refers to) in a cross-sectional view.

### BRIEF DESCRIPTION OF FIGURES

Embodiments of the invention will be discussed in conjunction with the accompanying drawings. Therein,
- Fig. 1: shows a schematic perspective view of a Raman spectroscopy apparatus including a Raman spectroscopy probe;
- Fig. 2: shows a cross-sectional view of the Raman spectroscopy probe according to Fig. 1;
- Fig. 3: shows a plan view on a distal end of the Raman spectroscopy probe according to Fig. 1;
- Fig. 4: shows a schematic perspective view of a second embodiment of a Raman spectroscopy apparatus including of a Raman spectroscopy probe;
- Fig. 5: shows a cross-sectional view of the Raman spectroscopy probe according to Fig. 4;
- Fig. 6: shows a plan view on a distal end of the Raman spectroscopy probe according to Fig. 4;
- Fig. 7: shows a cross-sectional view of a magnification layer;
- Fig. 8: shows a top view of the magnification layer of Fig. 7; and
- Fig. 9: shows a cross-sectional view of a second embodiment of a magnification layer.

### DESCRIPTION OF SOME EMBODIMENTS OF THE INVENTION

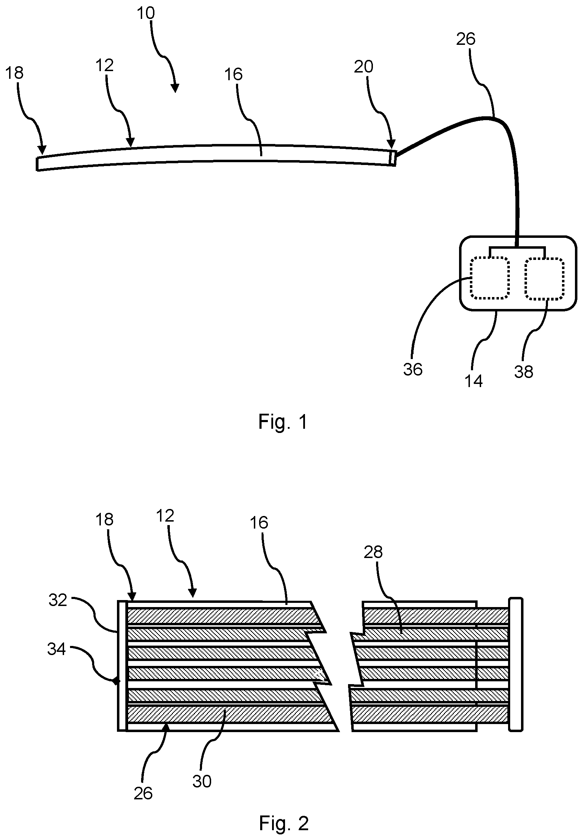

Fig. 1 shows a Raman spectroscopy apparatus 10 which includes a Raman spectroscopy probe 12 and/or an analysis device 14. The Raman spectroscopy probe 12 includes an elongate body 16 which extends from a distal end 18 to a proximal end 20. The Raman spectroscopy probe 12, and in particular the elongate body 16, has an elongated structure and is configured to be inserted into a cavity of a body. The Raman spectroscopy probe 12 may be an endoscopic device and can be bent or flexed in order to navigate the distal end 18 through the cavity of the body to a target site or target area. The target site may be a region within the cavity of the body (for example the lung) which is intended to be analysed, monitored and/or treated. In particular, tissue at the target site can be analysed using Raman spectroscopy.

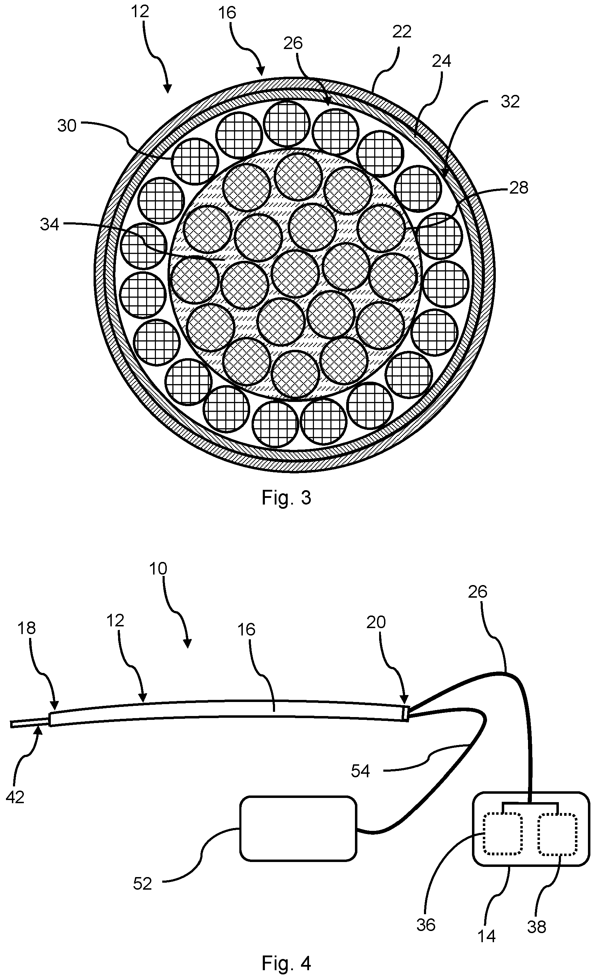

The elongate body 16 covers the Raman spectroscopy probe 12 from the distal end 18 to the proximal end 20. In particular, the elongate body 16 insulates the inside of the Raman spectroscopy probe 12 from its surrounding, for example from fluids and the like. As better visible in Fig. 3, the elongate body 16 includes an outer sheath 22 and a strain relief layer 24. The outer sheath 22 may be made from a lubricous polymer in order to reduce the friction when inserting the Raman spectroscopy probe 12 into the cavity of the body or scoping device such as a catheter. The strain relief layer 24 may include a braid, a coil, and/or tube made of a polymer or metal wire which each or in combination limit the angle of flexion of the Raman spectroscopy probe 12 such that components arranged within the elongate body 16 (to be described later) are not damaged when flexed or bent.

The Raman spectroscopy probe 12 further includes an optical transmission line 26 which may include at least one illumination fibre 28 and/or at least one collection fibre 30. In the embodiment shown in the figures, a plurality of illumination fibres 28 and collection fibres 30 are provided. As visible in Figs. 2 and 3, the plurality of collection fibre 30 forms a single ring of fibres while the illumination fibres 28 are position within the ring of collection fibres 30. The illumination fibres 28 and the collection fibres 30 may contact each other and are in contact with the elongate body 16, in particular the strain relief layer 24. However, the invention is not limited thereto. The illumination fibres 28 and/or the collection fibres 30 may not be in contact with each other or with the elongate body 16 but are arranged spaced apart.

The space between the illumination fibres 28 and collection fibres 30 may be filled with a filler material and/or an adhesive such as epoxy. The adhesive may be used to fixate the plurality of illumination fibres 28 and collection fibres 30 with respect to each other.

As visible in Fig. 2, the plurality of illumination fibres 28 and collection fibres 30 may extend beyond the proximal end 20 of the elongate body 16 and can be connected to the analysis device 14. A distal end of the illumination fibres 28 and the collection fibres 30 may coincide with the distal end 18 of the elongate body 16. In order to seal the distal end 18, a disc 32 may be provided at the distal end 18. The disc 32 may be in contact with the distal ends of the illumination fibres 28 and/or the collection fibres 30. The disc 32 is made from an optically transparent material such as fused silica, magnesium fluoride, and/or sapphire. The light propagating in the illumination fibres 28 and/or the collection fibres 30 passes through the disc 32. The disc may be a distal end surface of the at least one optical transmission line 26 and/or the elongate body 16.

The disc 32 may be a window through which light from the illumination fibres 28 can pass and/or light coming from the target site can be coupled into the collection fibres 30. The illumination fibres 28 and/or the collection fibre 30 may contact the disc 32 and/or can be spaced apart to the disc 32. The illumination fibres 28 and/or the collection fibres 30 may be fixedly positioned with respect to the disc 32.

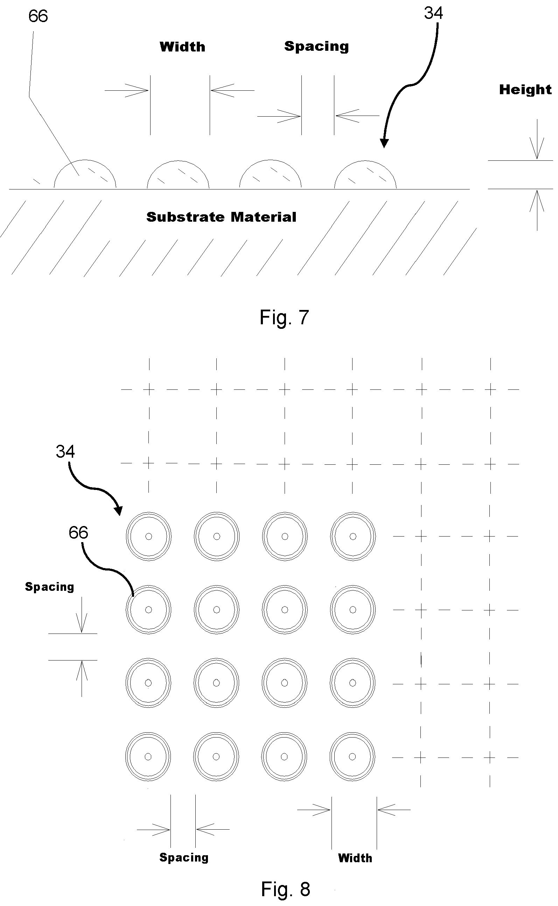

The disc 32 may include a magnification layer 34 on its outer surface. Thus, the magnification layer 34 is exposed or includes an exposed surface that can be brought in contact with the tissue at the target site. The disc 32 may be considered a substrate for the magnification layer 34. The magnification layer 34 may only be arranged in an area that is defined by the illumination fibres 28 (see dashed area in Fig. 3). The magnification layer 34 may not cover distal ends of the collection fibres 30. Thus, the magnification layer 34 may be arranged within the ring of collection fibres 30 and does not overlap with an area that is defined by distal ends of the collection fibres 30. Light exiting the illumination fibres 28 at their distal end passes through the magnification layer 34. Details of the magnification layer 34 are described later in conjunction with Figs. 7 to 9.

The plurality of illumination fibres 28 and collection fibres 30 forms a probe for Raman spectroscopy measurements. As commonly known, Raman spectroscopy includes illuminating a sample with monochromatic light while the inelastically back scattered light (or Raman scattered light) is analysed, for example using a spectrometer. The Raman scattered light has a different wavelength compared to the monochromatic light with which the sample is illuminated.

In the present case, the analysis device 14 includes a Raman light source 36 (such as one or more lasers) to which the illumination fibres 28 are coupled. Thus, the monochromatic light is directed to the target site via the illumination fibres 28. The Raman scattered light (the light inelastically scattered back by tissue in the target site) is coupled into the collection fibres 30 and fed to a spectrometer 38 arranged in the analysis device 14. The Raman scattered light is analysed using the spectrometer 38. The intensity and/or wavelength of the Raman scattered light is indicative of the molecules present within the tissue in the target site. The analysis of the molecules identified using the spectrometer 38 may indicate the presence of cancerous or other degenerated tissue at the target site. It is also possible to use the Raman spectroscopy measurements to identified different types of tissues present at the target site. This analysis step may be done manually or by a processor in combination with a respective software which may also be included in the analysis device 14.

In an embodiment not shown in the figures, the magnification layer 34 may be directly attached to distal ends of the illumination fibres 28.

Figs. 4 to 6 refer to another embodiment of the Raman spectroscopy apparatus 10 having the same features and characteristics as the embodiment depicted in Figs. 1 to 3 except of the following differences.

The Raman spectroscopy probe 12 further includes an instrument lumen 40 which extends from the distal end 18 to the proximal end 20 and is arranged within the elongate body 16. The instrument lumen 40 may be considered a passageway in which an elongate instrument 42 can be slidably or fixedly arranged. In other words, the elongate instrument 42 can be inserted into or pulled out of the instrument lumen 40. An inner diameter of the instrument lumen 40 is slightly greater than an outer diameter of the elongate instrument 42.

The instrument lumen 40 may be delimited by a hollow tube 44 which extends from the distal end 18 to the proximal end 20 and acts as a wall to seal the Raman spectroscopy probe 12, for example from fluids entering the space between the hollow tube 44 and the elongate instrument 42. The hollow tube 44 may be made from a polymer or other types of plastic materials, for example from the same material as the sheath 22. The lubricous polymer from which the hollow tube 44 may be made can contribute to the reduction of the friction between the elongate instrument 42 and the hollow tube 44.

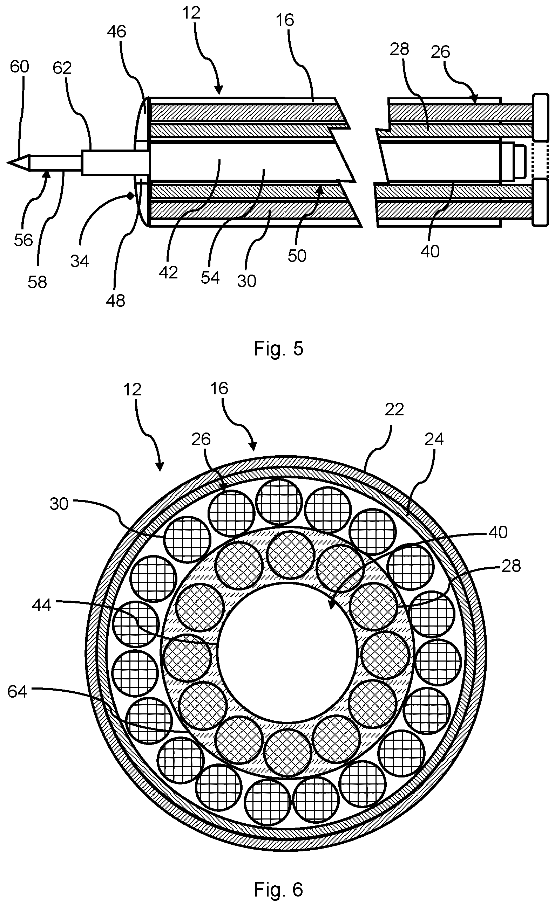

As better visible in Fig. 6, the instrument lumen 40 and, thus, the hollow tube 44 are coaxially arranged with the elongate body 16. Optionally, the instrument lumen 40 and/or the hollow tube 44 are centrally arranged within the elongate body 16. However, the invention is not limited to this embodiment. The instrument lumen 40 is arranged inside the elongate body 16 while the at least one illumination fibre 28 and at least one collection fibre 30 are arranged outside of the instrument lumen 40, in particular between the instrument lumen 40 and the elongate body 16.

**In** this embodiment of the Raman spectroscopy probe 12, a lens structure 46 is provided instead of the disc 32. The lens structure 46 may be coupled to the illumination fibres 28 and/or the collection fibres 30. The lens structure 46 may be a window through which light from the illumination fibres 28 can pass and/or light coming from the target site can be coupled into the collection fibres 30. The illumination fibres 28 and/or the collection fibre 30 may contact the lens structure 46 and/or can be spaced apart to the lens structure 46. The illumination fibres 28 and/or the collection fibres 30 may be fixedly positioned with respect to the lens structure 46.

The lens structure 46 may include one or more lenses and/or one or more optical filters. The one or more lenses may be configured to focus the light coming from the illumination fibres 28 onto the target site. The one or more lenses may also be configured to focus the light coming from the target site onto a distal end face of the collection fibres 30 such that the light may be coupled into the collection fibres 30. The one or more optical filters may be positioned before the distal end face of the collection fibres 30 and may be configured to filter out light having a wavelength identical to the light emitted by the illumination fibres 28, i.e. Rayleigh or elastically scattered light.

The magnification layer 34 may be arranged on an outer surface of the lens structure 46 such that the magnification layer 34 can be brought in contact with the tissue. The lens structure 46 or the part of the lens structure 46 on which the magnification layer 34 is placed can be made from an optically transparent material such as fused silica, magnesium fluoride, glass, and/or sapphire. The light propagating in the illumination fibres 28 and/or the collection fibres 30 passes through the lens structure 46.

The lens structure 46 may include an aperture 48 which is aligned with the instrument lumen 40 such that the elongate instrument 42 can be pushed out of the instrument lumen 40 and through the aperture 48 of the lens structure 46.

The plurality of illumination fibres 28 is arranged in a first ring of fibres, while the plurality of collection fibres 30 is arranged in a second ring of fibres. In each ring, the plurality of collection fibres 30 and illumination fibres 28 are densely packed and may be contact each other. The first ring of collection fibres 30 is coaxially arranged with the second ring of illumination fibres 28 and, optionally, coaxially with the instrument lumen 40 and/or the elongate body 16. As depicted in Fig. 6, the second ring of collection fibres 30 is separated by the first ring of illumination fibres 28 by a middle wall 64. However, the middle wall 64 is not essential and can be omitted. The middle wall 64 may be constituted by a hollow tube and can be made from a plastic material. The middle wall 64 may be coaxially arranged with the instrument lumen 40, the hollow tube 44 and/or the elongate body 16.

The second ring of collection fibres 30 surrounds the first ring of illumination fibres 28. Thus, the cross-sectional area covered by the collection fibres 30 is greater than a cross-sectional area covered by the illumination fibres 28. This may contribute to a collection of as much as Raman scattered light from the target site. For example, this arrangement results in that the number of collection fibres 30 is greater than the number of illumination fibres 28. Alternatively or additionally, the diameter of the collection fibres 30 may be greater than the diameter of the illumination fibres 28.

The magnification layer 34 may only cover the area of the first ring of illumination fibres 28 while the second ring of collection fibres 30 is not covered with the magnification layer 34.

The elongate instrument 42 of the embodiment depicted in Figs. 4 to 6 is an electrosurgical treatment device 50 for emitting electromagnetic radiation such as a radio frequency radiation and/or microwave radiation which may be used for cutting, ablating, stimulating and/or coagulating tissue at the target site.

A proximal end of the treatment device 50 may be connected to a generator 52 which is capable of generating electromagnetic energy having frequencies in the radio frequency range and/or the microwave range.

The treatment device 50 may include a flexible transmission line 54 (such as a coaxial cable) and a radiating element 56 which is connected at a distal end of the transmission line 54. The transmission line 54 may be a conventional flexible 50 Ω coaxial cable suitable for conveying microwave energy. The transmission line 54 may include a centre conductor and an outer conductor that are separated by a dielectric material. The transmission line 54 is connectable at a proximal end to a generator, e.g. the generator 52, to receive the electromagnetic energy, in particular microwave energy.

The radiating element 56 includes a proximal coaxial transmission line 58 and a distal needle tip 60 formed at a distal end of the proximal coaxial transmission line 58. The proximal coaxial transmission line 58 is electrically connected to the distal end of the transmission line 54 to receive the electromagnetic energy from the transmission line 54 and convey it to the distal needle tip 60. The distal needle tip 60 is configured to deliver the received electromagnetic energy into biological tissue at the target site. In the present example, the distal needle tip 60 is configured as transformer, such as a half wavelength transformer, to deliver microwave energy into target biological tissue, to ablate the target tissue. In other words, an electrical length of the distal needle tip 60 corresponds to a half wavelength of the microwave energy (e.g. at 5.8 GHz). When microwave energy is delivered to the distal needle tip 60, it may radiate the microwave energy along its length into surrounding biological tissue.

An inner conductor of the proximal coaxial transmission line 58 is electrically connected to the centre conductor of the transmission line 54. The radiating element 56 is secured to the transmission line 54 via a collar 62 mounted over a junction between the transmission line 54 and the radiating element 56. The collar 62 is made of a conductive material (e.g. brass), and electrically connects the outer conductor of the transmission line 54 to an outer conductor of the proximal coaxial transmission line 58. The outer conductor is formed of a tube of nitinol, which is flexible and provides a sufficient longitudinal rigidity to pierce tissue (e.g. the duodenum wall). For illustration purposes, the outer conductor is omitted from Fig. 6. Also for illustration purposes, a length of the proximal coaxial transmission line 58 has been omitted in Fig. 6. Exemplary details of the transmission line 54 and the radiating element 54 may be gathered from WO 2020/221749.

The transmission line 54 may be fixedly arranged within the instrument lumen 40. For example, the transmission line 54 is adhered by means of an adhesive to the hollow tube 44. However, the hollow tube 44 may be omitted in the embodiment depicted in Figs. 4 to 6 since a sealing of the instrument lumen 40 is no longer necessary as the transmission line 54 is arranged within the elongate body 16. For example, an outer sheath of the transmission line 54 may replace the hollow tube 44. The plurality of illumination fibres 28 and collection fibres 30 may be fixed to the transmission line 54.

The elongate instrument 42 may be an endoscopic camera (not shown in the Figs). The endoscopic or elongate camera may include an image capturing device. The image capturing device is configured to create a series of the electrical signals based on optical image which is projected on an optical sensor arranged within the image capturing device. The optical sensor may be arranged at or close to a distal end of the elongate camera. A wire may extend within the elongate camera from image capturing device (in particular the optical sensor) to a proximal end of the elongate camera for transmitting the electrical signals. The proximal end of the elongate camera is connected to a display device. The display device may include a processor to process the series of electrical signals generated by the image capturing device. In particular, the processor may generate an optical image that can be displayed by a display of the display device.

Figs. 7 to 9 depict two embodiments of the magnification layer 34 which can be applied to the disc 32 and/or the lens structure 46.

As visible in Figs. 7 and 8, the magnification layer 34 includes material patches 66 which are spaced apart from each other. The material patches 66 can be regarded as islands within the magnification layer 34. The material patches 66 are periodically arranged. The unit cell of the periodical arrangement of the material patches 66 is a square (see Fig. 8). A distance between two adjacent material patches 66 is between 10 nm to 120nm. The spacing between two adjacent material patches 66 in each direction is 40 nanometres in the embodiment depicted in Figs. 7 and 8.

The material patches 66 have a dome shape and have a height between 10 nm to 120nm, optionally 40 nm, and width (diameter) between 20 nm to 150nm, optionally 80 nm. The height can be measured from a substrate, which correspond to the disc 32 or the lens structure 46, to the highest point of the material patch 66. The material patches 66 can be made from metal, optionally gold, copper, silver, and/or aluminium.

Light can travel through the magnification layer 34 by passing through the free space between the material patches 66. Surface plasmons can be excited within the material patches 66 to provide the surface enhanced Raman scattering.

The material patches 66 do not need to be periodically arranged as depicted in Figs. 7 and 8. In addition, it is possible that the material patches 66 differ from each other in their shape and/or dimensions. This means that both the positioning and the shape of the material patches 66 may be randomly distributed throughout the magnification layer 34. The distance between two material patches 66 as well as the dimensions of the material patches 66 may be described by using an average value and a variation (variance) thereof.

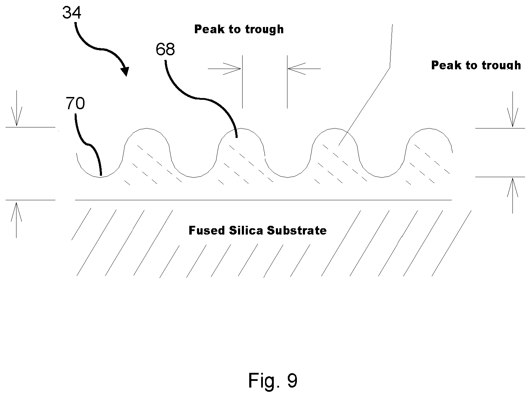

The embodiment of the magnification layer 34 as depicted in Fig. 9 is a continuous layer having protrusions 68 (which correspond to thick portions of the magnification layer 34) and recesses 70 (which correspond to thin portions of the magnification layer 34). The protrusions 68 and the recesses 70 may form a periodical grid extending in two dimensions. However, one dimensional grids of protrusions 68 and recesses 70 are also possible. Finally, it is also possible that the protrusions 68 and recesses 70 may be randomly arranged. The protrusions 68 may be considered corresponding to the material patches 66 whereas the recesses 70 may be considered corresponding to the free space between the material patches 66 (the recesses 70 having a thickness of zero).

The thickness of the recesses 70 may be measured from the substrate and may be in such a range that light exiting the illumination fibre 28 can pass through the recesses 70. The recesses 70 may have a thickness between 1 nm and 40 nm, optionally 20 nm.

The spacing between a peak of the protrusion 68 and a trough of the recess 70 may be between 10 nm and 120 nm, optionally 60 nm. The height between a peak of the protrusion 68 and a trough of the recess 70 may be between 10 nm and 120 nm, optionally 60 nm. The peaks of the protrusions 68 may be between 10 nm and 120 nm, optionally 80 nm, after the surface of the substrate.

## Claims

1. A Raman spectroscopy probe, comprising
an elongate body having a proximal end and a distal end,
an optical transmission line within the elongate body for guiding light for inducing Raman scattering in a tissue between the proximal end and the distal end in a distal direction and for guiding Raman scattered light between the distal end and the proximal end in a proximal direction, and
a magnification layer arranged at or on the distal end, the magnification layer having an exposed surface for contacting the tissue,
wherein the magnification layer is positioned such that the light for inducing Raman scattering impinges on the magnification layer,
wherein the magnification layer is for inducing surface-enhanced Raman scattering (SERS) at the exposed surface,
wherein the optical transmission line includes at least one illumination fibre for guiding light from the proximal end to the distal end and at least one collection fibre for guiding light from the distal end to the proximal end, and
wherein the magnification layer is positioned in optical communication with the at least one illumination fibre **characterised in that** the magnification layer is positioned not in optical communication with the at least one collection fibre.

2. The Raman spectroscopy probe according to claim 1, wherein the optical transmission line includes a distal end surface, wherein the magnification layer is arranged on the distal end surface.

3. The Raman spectroscopy probe according to claim 2, wherein the distal end surface includes a disk for closing the distal end.

4. The Raman spectroscopy probe according to claim 2, wherein the distal end surface is a distal end of the illumination fibre.

5. The Raman spectroscopy probe according to claim 1, further comprising a lens structure arranged at the distal end for focussing the light from the optical transmission line onto tissue contacting the exposed surface and/or for focussing the Raman scattered light from tissue contacting the exposed surface into the optical transmission line, wherein the magnification layer is arranged on the lens structure.

6. The Raman spectroscopy probe according to any preceding claim, wherein the magnification layer is made from an electrically conductive material and the exposed surface includes a surface structure on a nanoscale for inducing surface-enhanced Raman scattering.

7. The Raman spectroscopy probe according to any preceding claim, wherein the magnification layer includes material patches which are distributed over the magnification layer.

8. The Raman spectroscopy probe according to claim 7, wherein dimensions of the material patches and dimensions of spaces between the material patches are in the same order of magnitude.

9. The Raman spectroscopy probe according to claim 7 or 8, wherein the material patches have a width between 20 nm to 150 nm and/or a height between 10 nm to 120 nm, wherein optionally a distance between two adjacent material patches is between 10 nm to 120 nm.

10. The Raman spectroscopy probe according to any one of the claims 7 to 9, wherein the material patches are identical to each other, wherein optionally the material patches are dome shaped.

11. The Raman spectroscopy probe according to any one of the claims 1 to 6, wherein the magnification layer is a continuous material layer including thick portions defining protrusions and thin portions defining recesses, wherein the thin portions have a thickness which allows transmission of the light for inducing Raman scattering.

12. The Raman spectroscopy probe according to claim 11, wherein the thick portions have a height between 10 nm to 120 nm and/or the thin portions have a thickness between 1 nm and 40 nm.

13. The Raman spectroscopy probe according to any preceding claim, wherein the magnification layer includes a metal, in particular gold, silver, and/or aluminium.

14. The Raman spectroscopy probe according to claim 3 or 5, wherein the disk or the lens structure includes an optically transparent material, in particular fused silica, magnesium fluoride, and/or sapphire.

15. A Raman spectroscopy apparatus, comprising
the Raman spectroscopy probe according to any preceding claim and
an analysis device including a spectrometer and a Raman light source for generating monochromatic light,
wherein the optical transmission line is in optical communication with the analysis device, in particular to the Raman light source and/or the spectrometer.

## Patentansprüche

1. Raman-Spektroskopiesonde, umfassend:
einen länglichen Körper, der ein proximales Ende und ein distales Ende aufweist,
eine optische Übertragungsleitung innerhalb des länglichen Körpers zum Leiten von Licht zum Auslösen einer Raman-Streuung in einem Gewebe zwischen dem proximalen Ende und dem distalen Ende in eine distale Richtung und zum Leiten von Raman-gestreutem Licht zwischen dem distalen Ende und dem proximalen Ende in eine proximale Richtung und
eine Vergrößerungsschicht, die an oder auf dem distalen Ende angeordnet ist, wobei die Vergrößerungsschicht eine freigelegte Oberfläche zum Kontakt mit dem Gewebe aufweist,
wobei die Vergrößerungsschicht so angeordnet ist, dass das Licht zum Auslösen einer Raman-Streuung auf die Vergrößerungsschicht auftrifft,
wobei die Vergrößerungsschicht zum Auslösen einer oberflächenverstärkten Raman-Streuung (SERS) an der freigelegten Oberfläche dient,
wobei die optische Übertragungsleitung zumindest eine Beleuchtungsfaser zum Leiten von Licht von dem proximalen Ende an das distale Ende und zumindest eine Bündelungsfaser zum Leiten von Licht von dem distalen Ende an das proximale Ende umfasst und
wobei die Vergrößerungsschicht in optischer Kommunikation mit der zumindest einen Beleuchtungsfaser angeordnet ist, **dadurch gekennzeichnet, dass** die Vergrößerungsschicht nicht in optischer Kommunikation mit der zumindest einen Bündelungsfaser angeordnet ist.

2. Raman-Spektroskopiesonde nach Anspruch 1, wobei die optische Übertragungsleitung eine distale Endoberfläche umfasst, wobei die Vergrößerungsschicht auf der distalen Endoberfläche angeordnet ist.

3. Raman-Spektroskopiesonde nach Anspruch 2, wobei die distale Endoberfläche eine Scheibe zum Verschließen des distalen Endes umfasst.

4. Raman-Spektroskopiesonde nach Anspruch 2, wobei die distale Endoberfläche ein distales Ende der Beleuchtungsfaser ist.

5. Raman-Spektroskopiesonde nach Anspruch 1, die weiters eine Linsenstruktur umfasst, die an dem distalen Ende angeordnet ist, zum Fokussieren von Licht aus der optischen Übertragungsleitung auf Gewebe, das die freigelegte Oberfläche kontaktiert, und/oder zum Fokussieren von Raman-gestreutem Licht aus Gewebe, das die freigelegte Oberfläche kontaktiert, in die optische Übertragungsleitung, wobei die Vergrößerungsschicht auf der Linsenstruktur angeordnet ist.

6. Raman-Spektroskopiesonde nach einem der vorangegangenen Ansprüche, wobei die Vergrößerungsschicht aus einem elektrisch leitenden Material besteht und die freigelegte Oberfläche eine Oberflächenstruktur auf Nanoskala zum Auslösen einer oberflächenverstärkten Raman-Streuung umfasst.

7. Raman-Spektroskopiesonde nach einem der vorangegangenen Ansprüche, wobei die Vergrößerungsschicht Materialstellen umfasst, die über die Vergrößerungsschicht verteilt sind.

8. Raman-Spektroskopiesonde nach Anspruch 7, wobei Abmessungen der Materialstellen und Abmessungen von Abständen zwischen den Materialstellen in derselben Größenordnung liegen.

9. Raman-Spektroskopiesonde nach Anspruch 7 oder 8, wobei die Materialstellen eine Breite von zwischen 20 nm und 150 nm und/oder eine Höhe von zwischen 10 nm bis 120 nm aufweisen, wobei ein Abstand zwischen zwei benachbarten Materialstellen gegebenenfalls zwischen 10 nm bis 120 nm liegt.

10. Raman-Spektroskopiesonde nach einem der Ansprüche 7 bis 9, wobei die Materialstellen miteinander identisch sind, wobei die Materialstellen gegebenenfalls kuppelförmig sind.

11. Raman-Spektroskopiesonde nach einem der Ansprüche 1 bis 6, wobei die Vergrößerungsschicht eine kontinuierliche Materialschicht, einschließlich dicker Abschnitte, die Ausbuchtungen definieren, und dünner Abschnitte, die Vertiefungen definieren, ist, wobei die dünnen Abschnitte eine Dicke aufweisen, die eine Übertragung des Lichts zum Auslösen einer Raman-Streuung ermöglicht.

12. Raman-Spektroskopiesonde nach Anspruch 11, wobei die dicken Abschnitte eine Höhe von zwischen 10 nm bis 120 nm aufweisen und/oder die dünnen Abschnitte eine Dicke von zwischen 1 nm und 40 nm aufweisen.

13. Raman-Spektroskopiesonde nach einem der vorangegangenen Ansprüche, wobei die Vergrößerungsschicht ein Metall, insbesondere Gold, Silber und/oder Aluminium, umfasst.

14. Raman-Spektroskopiesonde nach Anspruch 3 oder 5, wobei die Scheibe oder die Linsenstruktur ein optisch transparentes Material, insbesondere Quarzglas, Magnesiumfluorid und/oder Saphir, umfasst.

15. Raman-Spektroskopievorrichtung, umfassend:
eine Raman-Spektroskopiesonde nach einem der vorangegangenen Ansprüche und
eine Analysevorrichtung, umfassend ein Spektrometer und eine Raman-Lichtquelle zum Erzeugen von monochromatischem Licht,
wobei die optische Übertragungsleitung in optischer Kommunikation mit der Analysevorrichtung, insbesondere mit der Raman-Lichtquelle und/oder dem Spektrometer, steht.

## Revendications

1. Sonde de spectroscopie Raman, comprenant
un corps allongé présentant une extrémité proximale et une extrémité distale,
une ligne de transmission optique à l'intérieur du corps allongé pour guider de la lumière afin d'induire une diffusion par effet Raman dans un tissu entre l'extrémité proximale et l'extrémité distale dans une direction distale et de guider de la lumière diffusée par effet Raman entre l'extrémité distale et l'extrémité proximale dans une direction proximale, et
une couche d'amplification agencée au niveau ou sur l'extrémité distale, la couche d'amplification présentant une surface exposée destinée à venir en contact avec le tissu,
dans laquelle la couche d'amplification est positionnée de telle sorte que la lumière pour induire une diffusion par effet Raman heurte la couche d'amplification,
dans laquelle la couche d'amplification est destinée à induire une diffusion par effet Raman améliorée en surface (SERS) au niveau de la surface exposée,
dans laquelle la ligne de transmission optique comprend au moins une fibre d'éclairage pour guider de la lumière de l'extrémité proximale à l'extrémité distale et au moins une fibre de collecte pour guider de la lumière de l'extrémité distale à l'extrémité proximale, et
dans laquelle la couche d'amplification est positionnée en communication optique avec la au moins une fibre d'éclairage, **caractérisée en ce que** la couche d'amplification n'est pas positionnée en communication optique avec la au moins une fibre de collecte.

2. Sonde de spectroscopie Raman selon la revendication 1, dans laquelle la ligne de transmission optique inclut une surface d'extrémité distale, dans laquelle la couche d'amplification est agencée sur la surface d'extrémité distale.

3. Sonde de spectroscopie Raman selon la revendication 2, dans laquelle la surface d'extrémité distale inclut un disque pour fermer l'extrémité distale.

4. Sonde de spectroscopie Raman selon la revendication 2, dans laquelle la surface d'extrémité distale est une extrémité distale de la fibre d'éclairage.

5. Sonde de spectroscopie Raman selon la revendication 1, comprenant en outre une structure de lentille agencée au niveau de l'extrémité distale pour focaliser la lumière provenant de la ligne de transmission optique sur un tissu venant en contact avec la surface exposée et/ou pour focaliser la lumière diffusée par effet Raman provenant d'un tissu venant en contact avec la surface exposée dans la ligne de transmission optique, dans laquelle la couche d'amplification est agencée sur la structure de lentille.

6. Sonde de spectroscopie Raman selon l'une quelconque des revendications précédentes, dans laquelle la couche d'amplification est fabriquée à partir d'un matériau électriquement conducteur et la surface exposée inclut une structure de surface à l'échelle nanométrique pour induire une diffusion par effet Raman améliorée en surface.

7. Sonde de spectroscopie Raman selon l'une quelconque des revendications précédentes, dans laquelle la couche d'amplification inclut des pastilles de matériau qui sont réparties sur la couche d'amplification.

8. Sonde de spectroscopie Raman selon la revendication 7, dans laquelle les dimensions des pastilles de matériau et les dimensions des espaces entre les pastilles de matériau sont du même ordre de grandeur.

9. Sonde de spectroscopie Raman selon la revendication 7 ou 8, dans laquelle les pastilles de matériau présentent une largeur comprise entre 20 nm et 150 nm et/ou une hauteur comprise entre 10 nm et 120 nm, dans laquelle facultativement une distance entre deux pastilles de matériau adjacentes est comprise entre 10 nm et 120 nm.

10. Sonde de spectroscopie Raman selon l'une quelconque des revendications 7 à 9, dans laquelle les pastilles de matériau sont identiques les unes aux autres, dans laquelle facultativement les pastilles de matériau sont en forme de dôme.

11. Sonde de spectroscopie Raman selon l'une quelconque des revendications 1 à 6, dans laquelle la couche d'amplification est une couche de matériau continue comprenant des parties épaisses définissant des saillies et des parties minces définissant des évidements, dans laquelle les parties minces présentent une épaisseur qui permet la transmission de la lumière pour induire une diffusion par effet Raman.

12. Sonde de spectroscopie Raman selon la revendication 11, dans laquelle les parties épaisses présentent une hauteur entre 10 nm et 120 nm et/ou les parties minces présentent une épaisseur entre 1 nm et 40 nm.

13. Sonde de spectroscopie Raman selon l'une quelconque des revendications précédentes, dans laquelle la couche d'amplification inclut un métal, en particulier de l'or, de l'argent et/ou de l'aluminium.

14. Sonde de spectroscopie Raman selon la revendication 3 ou 5, dans laquelle le disque ou la structure de lentille comprend un matériau optiquement transparent, en particulier de la silice fondue, du fluorure de magnésium et/ou du saphir.

15. Appareil de spectroscopie Raman, comprenant
la sonde de spectroscopie Raman selon l'une quelconque des revendications précédentes et
un dispositif d'analyse incluant un spectromètre et une source de lumière Raman pour générer une lumière monochromatique,
dans lequel la ligne de transmission optique est en communication optique avec le dispositif d'analyse, en particulier avec la source de lumière Raman et/ou le spectromètre.
